# EUROPEAN PATENT SPECIFICATION

(11) **EP 3 965 759 B1**
(45) Date of publication and mention of the grant of the patent: **12.02.2025**
(21) Application number: 20806518.5
(22) Date of filing: 07.04.2020
(51) Int. Cl.: A61K 31/4015, A61K 31/455, A61K 9/20, A61K 31/401, A61K 31/4422, A61K 38/05, A61K 38/55

(54) **A TABLET FORMULATION COMPRISING LERCANIDIPINE AND ENALAPRIL**
TABLETTENFORMULIERUNG MIT LERCANIDIPIN UND ENALAPRIL
FORMULATION DE COMPRIMÉ COMPRENANT DE LA LERCANIDIPINE ET DE L'ÉNALAPRIL

(30) Priority: 10.05.2019 TR 201907094
(43) Date of publication of application: 16.03.2022
(73) Proprietor: Sanovel Ilac Sanayi ve Ticaret A.S., Sisli - Istanbul (TR)
(72) Inventor: TOMBAYOGLU, Vildan, 34460 Sar yer/Istanbul (TR); PACALI, Mustafa, 34460 Sar yer/Istanbul (TR); PALANTOKEN, Arzu, 34460 Sar yer/Istanbul (TR); TURKYILMAZ, Ali, 34460 Sar yer/Istanbul (TR)
(74) Representative: Genç Ilhan, Oznur
(86) International application number: PCT/TR2020/050280
(87) International publication number: WO 2020/231367

(56) References cited:
- WO-A2-2011/161223
- WO-A2-2011/161223
- WO-A2-2012/085249
- CN-A- 101 612 403
- CN-A- 101 836 982
- CN-A- 103 239 708
- CN-A- 104 069 500
- US-A1- 2008 175 872

## Description

### Field of the Invention

The present invention relates to a tablet formulation according to claim 1. Further, the present invention provides a method for the preparation of a tablet composition, according to claim 12.

### Background of the Invention

Hypertension is one of the most common cardiovascular disease states. In the United States, over 50 million people have been diagnosed with hypertension (which is defined as a blood pressure greater than or equal to 140/90 mm Hg). Elevated arterial pressure can cause pathological changes in the vasculature and hypertrophy of the left ventricle. Due to the damage that can be produced by hypertension, it is proposed to be the principal cause of stroke, myocardial infarction, and sudden cardiac death. Additionally, it is believed to be a major contributor to cardiac failure, renal insufficiency, and dissecting aneurysm of the aorta. The renin-angiotensin system is an important regulator of arterial pressure. The inactive angiotensinogen peptide is converted to the pro-peptide angiotensin I by the enzyme renin. Angiotensin I then is converted to the active angiotensin II form by the angiotensin converting enzyme. Angiotensin II then acts through a variety of receptor mediated mechanisms, such as increasing the total peripheral resistance and inhibiting the excretion of sodium and water by the kidneys, to increase arterial pressure.

Angiotensin converting enzyme (ACE) inhibitors are active agents that prevent the conversion of angiotensin I into angiotensin II.

The chemical name of enalapril is ((S)-1-[N-1-(ethoxycarbonyl)-3-phenylpropyl]-L-alanyl-L-proline). It is a prodrug of the active ACE inhibitor and has a chemical structure which is shown in Formula I.

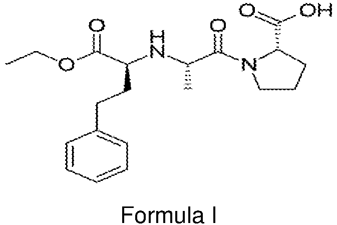

Enalapril is rapidly absorbed following oral administration with peak plasma levels of enalaprilat occurring in about 3 to 4 hours. The antihypertensive action of enalapril is believed to result primarily from the suppression of the renin-angiotensin system as a result of inhibition of angiotensin II formation. The recommended starting dose of enalapril as monotherapy is 5 mg daily, with drug titration 10 to 40 mg per day. The most common dosage is 20 mg per day.

Another class of active agents that are used for the treatment of hypertension is calcium antagonists. These active agents influence the influx of calcium ions into cells, especially smooth muscle cells. Inhibition of calcium influx produces a relaxation of smooth muscles, such as those around the arteries and veins, which leads to a decrease in observed hypertension. Such active agents as well as their hypotensive activity are described in a number of publications and patent applications.

The chemical name of lercanidipine is methyl-1,1-dimethyl-2-(N-(3,3-diphenylpropyl)-N-methylamino)ethyl-2,6-dimethyl-4-(3-nitrophenyl)-1,4-dihydropyridine-3,5-dicarboxylate and has a chemical structure which is shown in Formula II.

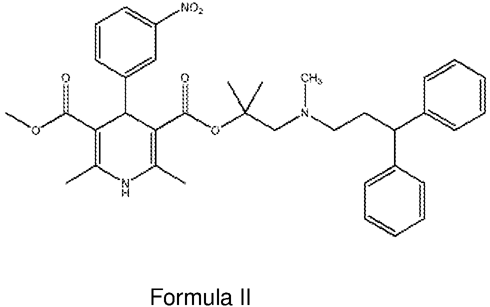

Lercanidipine is a highly lipophilic dihydropyridine calcium antagonist with a long duration of action and high vascular selectivity. Its mechanism of antihypertensive activity is due to a direct relaxant effect on vascular smooth muscle, thus lowering total peripheral resistance. The recommended starting dose of lercanidipine as monotherapy is 10 mg daily by the oral route, with a drug titration to 20 mg daily. It has been approved for the treatment of hypertension and has been marketed in several European countries under the trademark Zanidip^{®}.

Several pharmacological rationales have been used for combining an ACE-inhibitor and a calcium antagonist to treat hypertension. For example, the fact that multiple physiologic systems participate in blood pressure control has been proposed as the reason why individual active agents decrease in efficacy over time. A combination of treatments increases the number of mechanisms potentially capable of reducing elevated blood pressure and reduces the rate and magnitude of the adverse events produced by each drug. Further, the addition of one agent may counteract some deleterious effect of the other. Therefore, a low-dose combination of two different agents reduces the risk of dose-related adverse reaction while still allowing sufficient blood pressure reduction.

Disclosed are compositions and methods for treating hypertension comprising enalapril and lercanidipine in amounts effective in combination to reduce blood pressure to patents in need of treatment.

The fixed combination of lercanidipine HCl and enalapril maleate is marketed by Recordati and Berlin Chemie under the trade names Lercaril^{®}, Carmen ACE^{®}, and Zanipress^{®}. A tablet comprising a combination of lercanidipine hydrochloride and enalapril maleate is described in US 2003/0180355. It is explained in the description that solid unit dosage forms are prepared by mixing the active agents with the carrier and any other desired additives until a homogenous mixture is formed. Further tablet compositions comprising lercanidipine and enalapril are disclosed in WO 2011/161223 A2 and CN 103 239 708 A. US 2008/175872 A1 discloses a tablet comprising lercanidipine and colloidal silica, while CN 101 612 403 A discloses a capsule comprising enalapril and silica.

However, lercanidipine and enalapril are known to be susceptible to degradation and/or oxidation when subjected to unfavorable physical and/or chemical conditions.

There is thus still a need for a physically and chemically stable composition comprising lercanidipine and enalapril for effective combination anti-hypertensive treatments that have desired dissolution profile.

### Detailed Description of the Invention

The main object of the present invention is to avoid degradation and/or oxidation problems of active agents and to provide the desired compression and flowability of the formulation by using pharmaceutically acceptable excipients.

The other object of the present invention is to provide a desired stability.

The other object of the present invention is to provide the desired dissolution profile with an easy and cost-effective process for the preparation of the said pharmaceutical formulation. The term "lercanidipine" as used herein refers to lercanidipine in the form the free base or in the form of pharmaceutically acceptable salts, crystalline polymorph, solvates, hydrates, esters or mixture thereof.

The term "enalapril" as used herein refers to enalapril in the form the free base or in the form of pharmaceutically acceptable salts, crystalline polymorph, solvates, hydrates, esters or mixture thereof.

According to the present invention, lercanidipine hydrochloride and enalapril maleate are used.

According to the present invention, a tablet formulation comprises
∘ enalapril maleate
∘ lercanidipine hydrochloride
∘ at least one buffering agent
wherein further comprising at least two fillers and wherein said tablet formulation further comprises colloidal silicon dioxide as a glidant; wherein the total amount of enalapril maleate in the tablet is between 3.0% and 15.0% by weight, the total amount of lercanidipine hydrochloride in the tablet is between 1.0% and 8.0% by weight.

Suitable fillers are selected from the group comprising pregelatinized starch, lactose, ammonium alginate, calcium carbonate, calcium phosphate, calcium phosphate dihydrate, calcium sulfate, cellulose acetate, ethylcellulose, glyceryl palmitostearate, magnesium carbonate, magnesium oxide, maltodextrin, maltose, medium chain triglycerides, polymethacrylates, sodium alginate, erythritol, dibasic calcium phosphate, tribasic calcium phosphate, hydroxypropyl methylcellulose, sorbitol or mixtures thereof.

According to one embodiment of the present invention, the filler is pregelatinized starch and lactose.

In this present invention, it has been surprisingly found that using at least two fillers ensures compression and flowability of the formulation. Especially, using both pregelatinized starch and lactose provides desired compression and flowability of the formulation.

Lercanidipine and enalapril are sensitive to water and to oxidizing agents, so when including them into pharmaceutical formulations, care should be taken that the pharmaceutically acceptable excipient(s) used do not contain a non-negligible quantity of them. Especially, lercanidipine is pH sensitive and is more stable at acidic pH values, e.g. at pH values from 2 to 6, particularly from 3 to 5, as measured in a 20% (m/V) aqueous suspension. In the present invention, considering these properties of lercanidipine, the appropriate buffering agent is chosen at the appropriate rate in the formulation.

According to one embodiment of the present invention, the total amount of buffering agent in the tablet is between 1.0% and 20.0%, between 3.0% and 12.0%, preferably between 5.0% and 9.0% by weight.

According to one embodiment of the present invention, suitable buffering agents are selected from the group comprising sodium hydrogen carbonate, fumaric acid, sorbic acid, succinic acid, tartaric acid, propionic acid, alginic acid, sodium carbonate, sodium bicarbonate, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate or mixtures thereof.

According to one embodiment of the present invention, the buffering agent is sodium hydrogen carbonate to stabilize both enalapril maleate and lercanidipine hydrochloride. The process for increasing the stability of enalapril maleate against cyclization and/or hydrolysis comprises contacting enalapril maleate with a sufficient amount of sodium hydrogen carbonate.

According to the present invention, the total amount of enalapril maleate in the tablet is between 3.0% and 15.0%, preferably between 5.0% and 12.0% by weight.

According to the present invention, the total amount of lercanidipine hydrochloride in the tablet is between 1.0% and 8.0%, preferably between 2.0% and 6.0% by weight.

According to one embodiment of the present invention, the tablet formulation further comprises pharmaceutically acceptable excipients which are selected from the group comprising binders, disintegrants, glidants, lubricants or mixtures thereof.

Suitable binders are selected from the group comprising polyvinylpyrrolidone, alginates, carbomers, carboxymethylcellulose sodium, cellulose acetate phthalate, starch, corn starch, pregelatinized starch, starch mucilage, acacia mucilage, dextrates, dextrin, dextrose, ethylcellulose, glyceryl behenate, cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polydextrose, polyethylene oxide, polymethacrylates, alumina hydroxide, stearic acid, bentonite, cetostearyl alcohol, polyoxyethylene alkyl ethers or mixtures thereof.

According to one embodiment of the present invention, the binder is polyvinylpyrrolidone. Suitable disintegrants are selected from the group comprising sodium starch glycolate, low-substituted hydroxypropyl cellulose, sodium carboxymethyl cellulose, calcium carboxymethyl cellulose, carboxymethyl cellulose, docusate sodium, sodium lauryl sulfate, sodium alginate, magnesium aluminum silica, sodium glycine carbonate or mixtures thereof.

According to one embodiment of the present invention, the disintegrant is sodium starch glycolate.

According to the present invention, the composition comprises colloidal silicon dioxide as a glidant. Further suitable glidants may be selected from group comprising talc, aluminum silicate, calcium silicate, magnesium silicate, magnesium oxide, starch or mixtures thereof.

Suitable lubricants are selected from the group comprising magnesium stearate, calcium stearate, zinc stearate, boric acid, hydrogenated vegetable oil, sodium chlorate, magnesium lauryl sulfate, sodium oleate, sodium acetate, sodium benzoate, polyethylene glycol, glyceryl palmito sulfate, sodium stearyl fumarate, sodium lauryl sulfate or mixtures thereof.

According to one embodiment of the present invention, the lubricant is magnesium stearate. According to one embodiment of the present invention, the tablet formulation comprises enalapril maleate, lercanidipine hydrochloride, pregelatinized starch, sodium starch glycolate, polyvinylpyrrolidone, sodium hydrogen carbonate, lactose, silicon dioxide, magnesium stearate or mixtures thereof.

The tablet formulation of the present invention is prepared, using standard techniques and manufacturing processes well known in the art, such as direct compression, wet or dry granulation, hot melt granulation, hot melt extrusion, fluidized bed granulation, spray drying. According to one embodiment of the present invention, preferably, the tablet formulation of the present invention is prepared direct compression.

The method of preparation and type of excipients are selected to give the tablet formulation the desired physical characteristics that allow for the rapid compression of the tablets. After compression, the tablets have a number of additional attributes, such as appearance, hardness, disintegrating ability and an acceptable dissolution profile. Choice of fillers and other excipients depend on the chemical and physical properties of the drug, behavior of the mixture during processing and the properties of the final tablets.

### Example 1: A tablet formulation

| **Ingredients** | **Amount (% by weight of the total)** |
|---|---|
| Enalapril maleate | 3.0 - 15.0 |
| Lercanidipine hydrochloride | 1.0 - 8.0 |
| Pregelatinized starch | 12.0 - 25.0 |
| Sodium starch glycolate | 1.0 - 10.0 |
| Polyvinylpyrrolidone | 3.0 - 7.0 |
| Sodium hydrogen carbonate | 1.0 - 20.0 |
| Lactose | 40.0 - 55.0 |
| Silicon dioxide | 0.1 - 3.0 |
| Magnesium stearate | 0.1 - 3.0 |
| Coating | 1.0 - 5.0 |
| **Total** | **100** |

### Example 2: A tablet formulation

| **Ingredients** | **Amount (% by weight of the total)** |
|---|---|
| Enalapril maleate | 8.0 - 10.0 |
| Lercanidipine hydrochloride | 3.5 - 5.2 |
| Pregelatinized starch | 15.0 - 20.0 |
| Sodium starch glycolate | 4.5 - 6.2 |
| Polyvinylpyrrolidone | 3.5 - 5.2 |
| Sodium hydrogen carbonate | 5.0 - 10.0 |
| Lactose | 45.0 - 50.0 |
| Silicon dioxide | 0.1 - 2.0 |
| Magnesium stearate | 0.1 - 2.0 |
| Coating | 1.0 - 4.0 |
| **Total** | **100** |

### Example 3: A tablet formulation

| **Ingredients** | **Amount (% by weight of the total)** |
|---|---|
| Enalapril maleate | 9.76 |
| Lercanidipine hydrochloride | 4.88 |
| Pregelatinized starch | 18.8 |
| Sodium starch glycolate | 5.85 |
| Polyvinylpyrrolidone | 4.88 |
| Sodium hydrogen carbonate | 7.32 |
| Lactose | 46.49 |
| Silicon dioxide | 0.98 |
| Magnesium stearate | 0.98 |
| Coating | 2.5 |
| **Total** | **100** |

### Process for example 1 or 2 or 3:

The process for the preparation of the tablet formulation comprises the following steps:
a) mixing enalapril maleate, lercanidipine hydrochloride, pregelatinized starch, sodium starch glycolate, polyvinylpyrrolidone, sodium hydrogen carbonate and lactose
b) adding silicon dioxide and mixing
c) then, adding magnesium stearate and mixing
d) pressing to form a tablet
e) then, the tablets with a coating.

## Claims

1. A tablet formulation comprising;
- enalapril maleate
- lercanidipine hydrochloride
- at least one buffering agent
wherein further comprising at least two fillers and wherein said tablet formulation further comprises colloidal silicon dioxide as a glidant; wherein the total amount of enalapril maleate in the tablet is between 3.0% and 15.0% by weight, the total amount of lercanidipine hydrochloride in the tablet is between 1.0% and 8.0% by weight.

2. The tablet formulation according to claim 1, wherein said fillers are selected from the group comprising pregelatinized starch, lactose, ammonium alginate, calcium carbonate, calcium phosphate, calcium phosphate dihydrate, calcium sulfate, cellulose acetate, ethylcellulose, glyceryl palmitostearate, magnesium carbonate, magnesium oxide, maltodextrin, maltose, medium chain triglycerides, polymethacrylates, sodium alginate, erythritol, dibasic calcium phosphate, tribasic calcium phosphate, hydroxypropyl methylcellulose, sorbitol or mixtures thereof.

3. The tablet formulation according to claim 2, wherein the filler is pregelatinized starch and lactose.

4. The tablet formulation according to claim 1, wherein the total amount of buffering agent in the tablet is between 1.0% and 20.0%, preferably between 3.0% and 12.0% by weight.

5. The tablet formulation according to claim 4, wherein the buffering agents are selected from the group comprising sodium hydrogen carbonate, fumaric acid, sorbic acid, succinic acid, tartaric acid, propionic acid, alginic acid, sodium carbonate, sodium bicarbonate, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate or mixtures thereof.

6. The tablet formulation according to claim 1, wherein the tablet formulation further comprising pharmaceutically acceptable excipients which are selected from the group comprising binders, disintegrants, glidants, lubricants or mixtures thereof.

7. The tablet formulation according to claim 6, wherein the binders are selected from the group comprising polyvinylpyrrolidone, alginates, carbomers, carboxymethylcellulose sodium, cellulose acetate phthalate, starch, corn starch, pregelatinized starch, starch mucilage, acacia mucilage, dextrates, dextrin, dextrose, ethylcellulose, glyceryl behenate, cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polydextrose, polyethylene oxide, polymethacrylates, alumina hydroxide, stearic acid, bentonite, cetostearyl alcohol, polyoxyethylene alkyl ethers or mixtures thereof.

8. The tablet formulation according to claim 7, wherein the binder is polyvinylpyrrolidone.

9. The tablet formulation according to claim 6, wherein the disintegrants are selected from the group comprising sodium starch glycolate, low-substituted hydroxypropyl cellulose, sodium carboxymethyl cellulose, calcium carboxymethyl cellulose, carboxymethyl cellulose, docusate sodium, sodium lauryl sulfate, sodium alginate, magnesium aluminum silica, sodium glycine carbonate or mixtures thereof.

10. The tablet formulation according to claim 9, wherein the disintegrant is sodium starch glycolate.

11. The tablet formulation according to claim 6, wherein the tablet formulation comprises enalapril maleate, lercanidipine hydrochloride, pregelatinized starch, sodium starch glycolate, polyvinylpyrrolidone, sodium hydrogen carbonate, lactose, silicon dioxide and magnesium stearate.

12. A process for the preparing the tablet formulation according to claim 11, comprising the following steps:
a) mixing enalapril maleate, lercanidipine hydrochloride, pregelatinized starch, sodium starch glycolate, polyvinylpyrrolidone, sodium hydrogen carbonate and lactose
b) adding silicon dioxide and mixing
c) then, adding magnesium stearate and mixing
d) pressing to form a tablet
e) then, the tablets are coated.

## Patentansprüche

1. Tablettenformulierung, enthaltend;
- Enalaprilmaleat
- Lercanidipinhydrochlorid
- mindestens ein Puffermittel
wobei die Tablettenformulierung ferner mindestens zwei Füllstoffe umfasst und wobei die Tablettenformulierung ferner kolloidales Siliciumdioxid als Gleitmittel umfasst; wobei die Gesamtmenge an Enalaprilmaleat in der Tablette zwischen 3,0 und 15,0 Gew.-% liegt, die Gesamtmenge an Lercanidipinhydrochlorid in der Tablette zwischen 1,0 und 8,0 Gew.-% liegt.

2. Tablettenformulierung nach Anspruch 1, wobei die Füllstoffe aus der Gruppe ausgewählt sind, die vorverkleisterte Stärke, Laktose, Ammoniumalginat, Calciumkarbonat, Calciumphosphat, Calciumphosphatdihydrat, Calciumsulfat, Zelluloseacetat, Ethylzellulose, Glycerylpalmitostearat, Magnesiumcarbonat, Magnesiumoxid, Maltodextrin, Maltose, mittelkettige Triglyceride, Polymethacrylate, Natriumalginat, Erythrit, dibasisches Calciumphosphat, dreibasisches Calciumphosphat, Hydroxypropylmethylcellulose, Sorbit oder Mischungen davon umfasst.

3. Tablettenformulierung nach Anspruch 2, wobei der Füllstoff aus vorverkleisterter Stärke und Laktose besteht.

4. Tablettenformulierung nach Anspruch 1, wobei die Gesamtmenge des Puffermittels in der Tablette zwischen 1,0 und 20,0 Gew.-%, vorzugsweise zwischen 3,0 und 12,0 Gew.-% liegt.

5. Tablettenformulierung nach Anspruch 4, wobei die Puffermittel aus der Gruppe ausgewählt sind, die Natriumhydrogencarbonat, Fumarsäure, Sorbinsäure, Bernsteinsäure, Weinsäure, Propionsäure, Alginsäure, Natriumcarbonat, Natriumbicarbonat, Ascorbinsäure, Glutarsäure, Kaliumhydrogentartrat, Natriumhydrogentartrat, Kaliumhydrogenphthalat, Natriumhydrogenphthalat, Kaliumdihydrogenphosphat, Natriumdihydrogenphosphat, Dinatriumhydrogenphosphat oder Mischungen davon umfasst.

6. Tablettenformulierung nach Anspruch 1, wobei die Tablettenformulierung ferner pharmazeutisch akzeptable Hilfsstoffe umfasst, die aus der Gruppe ausgewählt sind, die Bindemittel, Sprengmittel, Gleitmittel, Schmiermittel oder Mischungen davon umfasst.

7. Tablettenformulierung nach Anspruch 6, wobei die Bindemittel aus der Gruppe ausgewählt sind, die Polyvinylpyrrolidon, Alginate, Carbomere, Carboxymethylcellulose-Natrium, Celluloseacetatphthalat, Stärke, Maisstärke, vorverkleisterte Stärke, Stärkeschleim, Gummiarabikumschleim, Dextrate, Dextrin, Dextrose, Ethylcellulose, Glycerylbehenat, Cellulose, Hydroxyethylmethylcellulose, Hydroxypropylcellulose, Hydroxypropylstärke, Hypromellose, flüssige Glucose, Magnesiumaluminiumsilicat, Maltodextrin, Maltose, Methylcellulose, Pektin, Poloxamer, Polydextrose, Polyethylenoxid, Polymethacrylate, Aluminiumhydroxid, Stearinsäure, Bentonit, Cetostearylalkohol, Polyoxyethylenalkylether oder Mischungen davon umfasst.

8. Tablettenformulierung nach Anspruch 7, wobei das Bindemittel Polyvinylpyrrolidon ist.

9. Tablettenformulierung nach Anspruch 6, wobei die Sprengmittel aus der Gruppe ausgewählt sind, die Natriumstärkeglykolat, niedrig substituierte Hydroxypropylcellulose, Natriumcarboxymethylcellulose, Calciumcarboxymethylcellulose, Carboxymethylcellulose, Docusat-Natrium, Natriumlaurylsulfat, Natriumalginat, Magnesiumaluminiumsiliciumdioxid, Natriumglycincarbonat oder Mischungen davon umfasst.

10. Tablettenformulierung nach Anspruch 9, wobei das Sprengmittel Natriumstärkeglykolat ist.

11. Tablettenformulierung nach Anspruch 6, wobei die Tablettenformulierung Enalaprilmaleat, Lercanidipinhydrochlorid, vorverkleisterte Stärke, Natriumstärkeglykolat, Polyvinylpyrrolidon, Natriumhydrogencarbonat, Lactose, Siliciumdioxid und Magnesiumstearat enthält.

12. Verfahren zur Herstellung der Tablettenformulierung nach Anspruch 11, umfassend die folgenden Schritte:
a) Mischung von Enalaprilmaleat, Lercanidipinhydrochlorid, Quellstärke, Natriumstärkeglykolat, Polyvinylpyrrolidon, Natriumhydrogencarbonat und Laktose
b) Zugabe von Siliciumdioxid und Mischen
c) dann Zugabe von Magnesiumstearat und Mischen
d) Pressen zur Tablettenform
e) Anschließend werden die Tabletten überzogen.

## Revendications

1. - Formulation de comprimé comprenant :
- du maléate d'énalapril ;
- du chlorhydrate de lercanidipine ;
- au moins un agent tampon,
comprenant en outre au moins deux charges et dans laquelle ladite formulation de comprimé comprend en outre du dioxyde de silicium colloïdal en tant qu'agent de glissement ; dans laquelle la quantité totale de maléate d'énalapril dans le comprimé est entre 3,0 % et 15,0 % en poids, la quantité totale de chlorhydrate de lercanidipine dans le comprimé est entre 1,0 % et 8,0 % en poids.

2. - Formulation de comprimé selon la revendication 1, dans laquelle lesdites charges sont choisies dans le groupe comprenant l'amidon prégélatinisé, le lactose, l'alginate d'ammonium, le carbonate de calcium, le phosphate de calcium, le phosphate de calcium dihydraté, le sulfate de calcium, l'acétate de cellulose, l'éthylcellulose, le palmitostéarate de glycéryle, le carbonate de magnésium, l'oxyde de magnésium, la maltodextrine, le maltose, les triglycérides à chaîne moyenne, les polyméthacrylates, l'alginate de sodium, l'érythritol, le phosphate de calcium dibasique, le phosphate de calcium tribasique, l'hydroxypropyl méthylcellulose, le sorbitol ou les mélanges de ceux-ci.

3. - Formulation de comprimé selon la revendication 2, dans laquelle la charge est de l'amidon prégélatinisé et du lactose.

4. - Formulation de comprimé selon la revendication 1, dans laquelle la quantité totale d'agent tampon dans le comprimé est entre 1,0 % et 20,0 %, de préférence entre 3,0 % et 12,0 % en poids.

5. - Formulation de comprimé selon la revendication 4, dans laquelle les agents tampons sont choisis dans le groupe comprenant l'hydrogéno carbonate de sodium, l'acide fumarique, l'acide sorbique, l'acide succinique, l'acide tartrique, l'acide propionique, l'acide alginique, le carbonate de sodium, le bicarbonate de sodium, l'acide ascorbique, l'acide glutarique, l'hydrogéno tartrate de potassium, l'hydrogéno tartrate de sodium, l'hydrogéno phtalate de potassium, l'hydrogéno phtalate de sodium, le dihydrogéno phosphate de potassium, le dihydrogéno phosphate de sodium, l'hydrogéno phosphate disodique ou les mélanges de ceux-ci.

6. - Formulation de comprimé selon la revendication 1, dans laquelle la formulation de comprimé comprend en outre des excipients pharmaceutiquement acceptables qui sont choisis dans le groupe comprenant les liants, les délitants, les agents de glissement, les lubrifiants ou des mélanges de ceux-ci.

7. - Formulation de comprimé selon la revendication 6, dans laquelle les liants sont choisis dans le groupe comprenant la polyvinylpyrrolidone, les alginates, les carbomères, la carboxyméthylcellulose sodique, l'acétate phtalate de cellulose, l'amidon, l'amidon de maïs, l'amidon prégélatinisé, le mucilage d'amidon, le mucilage d'acacia, les dextrates, la dextrine, le dextrose, l'éthylcellulose, le béhénate de glycéryle, la cellulose, l'hydroxyéthylméthyl cellulose, l'hydroxypropyl cellulose, l'hydroxypropyl amidon, l'hypromellose, le glucose liquide, le silicate d'aluminium et de magnésium, la maltodextrine, le maltose, la méthylcellulose, la pectine, le poloxamère, le polydextrose, le poly(oxyde d'éthylène), les polyméthacrylates, l'hydroxyde d'alumine, l'acide stéarique, la bentonite, l'alcool cétostéarylique, les éthers alkyliques polyoxyéthylénés ou les mélanges de ceux-ci.

8. - Formulation de comprimé selon la revendication 7, dans laquelle le liant est la polyvinylpyrrolidone.

9. - Formulation de comprimé selon la revendication 6, dans laquelle les délitants sont choisis dans le groupe comprenant le glycolate d'amidon sodique, l'hydroxypropyl cellulose faiblement substituée, la carboxyméthyl cellulose sodique, la carboxyméthyl cellulose de calcium, la carboxyméthyl cellulose, le docusate de sodium, le lauryl sulfate de sodium, l'alginate de sodium, la silice d'aluminium et de magnésium, le carbonate de glycine sodique ou les mélanges de ceux-ci.

10. - Formulation de comprimé selon la revendication 9, dans laquelle le délitant est le glycolate d'amidon sodique.

11. - Formulation de comprimé selon la revendication 6, dans laquelle la formulation de comprimé comprend du maléate d'énalapril, du chlorhydrate de lercanidipine, de l'amidon prégélatinisé, du glycolate d'amidon sodique, de la polyvinylpyrrolidone, de l'hydrogéno carbonate de sodium, du lactose, du dioxyde de silicium et du stéarate de magnésium.

12. - Procédé de préparation de la formulation de comprimé selon la revendication 11, comprenant les étapes suivantes :
a) mélanger le maléate d'énalapril, le chlorhydrate de lercanidipine, l'amidon prégélatinisé, le glycolate d'amidon sodique, la polyvinylpyrrolidone, l'hydrogéno carbonate de sodium et le lactose ;
b) ajouter le dioxyde de silicium et mélanger ;
c) puis, ajouter le stéarate de magnésium et mélanger ;
d) presser pour former un comprimé ;
e) ensuite, les comprimés sont enrobés.
